# EUROPEAN PATENT APPLICATION

(11) **EP 2 543 388 A1**
(43) Date of publication of application: **09.01.2013**
(21) Application number: 11750747.5
(22) Date of filing: 03.03.2011
(51) Int. Cl.: A61K 39/395, A61P 1/00, A61P 1/04

(54) **DRUG FOR INFLAMMATORY BOWEL DISEASE**

(30) Priority: 04.03.2010 JP 2010048052
(71) Applicant: Dainippon Sumitomo Pharma Co., Ltd., Chuo-ku Osaka-shi Osaka 541-0045 (JP)
(72) Inventor: WATANABE, Takamasa, Osaka-shi Osaka 554-0022 (JP)
(74) Representative: Vossius & Partner
(86) International application number: PCT/JP2011/054900
(87) International publication number: WO 2011/108638

(57) **Abstract**

Anti-CD81 antibody displays not only a remission induction effect, but also a long-term remission maintenance effect on inflammatory bowel disease when administered in a single dose, and is therefore effective as a drug for maintaining remission from inflammatory bowel disease. Moreover, anti-CD81 antibody is effective as a drug for the prevention, amelioration and treatment of inflammatory bowel disease because it has preventive, therapeutic and ameliorating effects on refractory inflammatory bowel disease.

SEQUENCE LISTING

<110> Dainippon Sumitomo Pharma Co., Ltd.

<120> Medicament for inflammatory bowel diseases

<130> W5991-000000

<160> 2

<170> PatentIn version 3.2

<210> 1
<211> 1497
<212> DNA
<213> Homo sapiens

<400> 1
gagcgagcgc gcaacggcgg cgacggcggc gaccccaccg cgcatcctgc caggcctccg 60
gcgcccagcg ccccacgcgc ccccgcgccc ccgcgccccc gcgccccttt cttcgcgccc 120
ccgcccctcg gcccgccagg cccccttgcc ggccacccgc caggccccgc gccggcccgc 180
ccgccgccca ggaccggccc gcgccccgca ggccgcccgc cgcccgcgcc gccatgggag 240
tggagggctg caccaagtgc atcaagtacc tgctcttcgt cttcaatttc gtcttctggc 300
tggctggagg cgtgatcctg ggtgtggccc tgtggctccg ccatgacccg cagaccacca 360
acctcctgta tctggagctg ggagacaagc ccgcgcccaa caccttctat gtaggcatct 420
acatcctcat cgctgtgggc gctgtcatga tgttcgttgg cttcctgggc tgctacgggg 480
ccatccagga atcccagtgc ctgctgggga cgttcttcac ctgcctggtc atcctgtttg 540
cctgtgaggt ggccgccggc atctggggct ttgtcaacaa ggaccagatc gccaaggatg 600
tgaagcagtt ctatgaccag gccctacagc aggccgtggt ggatgatgac gccaacaacg 660
ccaaggctgt ggtgaagacc ttccacgaga cgcttgactg ctgtggctcc agcacactga 720
ctgctttgac cacctcagtg ctcaagaaca atttgtgtcc ctcgggcagc aacatcatca 780
gcaacctctt caaggaggac tgccaccaga agatcgatga cctcttctcc gggaagctgt 840
acctcatcgg cattgctgcc atcgtggtcg ctgtgatcat gatcttcgag atgatcctga 900
gcatggtgct gtgctgtggc atccggaaca gctccgtgta ctgaggcccc gcagctctgg 960
ccacagggac ctctgcagtg ccccctaagt gacccggaca cttccgaggg ggccatcacc 1020
gcctgtgtat ataacgtttc cggtattact ctgctacacg tagccttttt acttttgggg 1080
ttttgttttt gttctgaact ttcctgttac cttttcaggg ctgacgtcac atgtaggtgg 1140
cgtgtatgag tggagacggg cctgggtctt ggggactgga gggcaggggt ccttctgccc 1200
tggggtccca gggtgctctg cctgctcagc caggcctctc ctgggagcca ctcgcccaga 1260
gactcagctt ggccaacttg gggggctgtg tccacccagc ccgcccgtcc tgtgggctgc 1320
acagctcacc ttgttccctc ctgccccggt tcgagagccg agtctgtggg cactctctgc 1380
cttcatgcac ctgtcctttc taacacgtcg ccttcaactg taatcacaac atcctgactc 1440
cgtcatttaa taaagaagga acatcaggca tgctaccagg cctgtgcagt ccctcag 1497

<210> 2
<211> 236
<212> PRT
<213> Homo sapiens

<400> 2

Met Gly Val Glu Gly Cys Thr Lys Cys Ile Lys Tyr Leu Leu Phe Val
1               5                   10                  15
Phe Asn Phe Val Phe Trp Leu Ala Gly Gly Val Ile Leu Gly Val Ala
            20                  25                  30
Leu Trp Leu Arg His Asp Pro Gln Thr Thr Asn Leu Leu Tyr Leu Glu
        35                  40                  45
Leu Gly Asp Lys Pro Ala Pro Asn Thr Phe Tyr Val Gly Ile Tyr Ile
    50                  55                  60
Leu Ile Ala Val Gly Ala Val Met Met Phe Val Gly Phe Leu Gly Cys
65                  70                  75                  80
Tyr Gly Ala Ile Gln Glu Ser Gln Cys Leu Leu Gly Thr Phe Phe Thr
                85                  90                  95
Cys Leu Val Ile Leu Phe Ala Cys Glu Val Ala Ala Gly Ile Trp Gly
            100                 105                 110
Phe Val Asn Lys Asp Gln Ile Ala Lys Asp Val Lys Gln Phe Tyr Asp
        115                 120                 125
Gln Ala Leu Gln Gln Ala Val Val Asp Asp Asp Ala Asn Asn Ala Lys
    130                 135                 140
Ala Val Val Lys Thr Phe His Glu Thr Leu Asp Cys Cys Gly Ser Ser
145                 150                 155                 160
Thr Leu Thr Ala Leu Thr Thr Ser Val Leu Lys Asn Asn Leu Cys Pro
                165                 170                 175
Ser Gly Ser Asn Ile Ile Ser Asn Leu Phe Lys Glu Asp Cys His Gln
            180                 185                 190
Lys Ile Asp Asp Leu Phe Ser Gly Lys Leu Tyr Leu Ile Gly Ile Ala
        195                 200                 205
Ala Ile Val Val Ala Val Ile Met Ile Phe Glu Met Ile Leu Ser Met
    210                 215                 220
Val Leu Cys Cys Gly Ile Arg Asn Ser Ser Val Tyr
225                 230                 235

## Description

### Technical Field

The present invention relates to a drug for maintaining remission of inflammatory bowel disease (hereinbelow, may be abbreviated as "IBD") that contains an anti-CD81 antibody as the active ingredient or a preventive, ameliorating, or therapeutic drug for refractory inflammatory bowel disease that contains an anti-CD81 antibody as the active ingredient. The present invention further relates to a method for maintaining remission of inflammatory bowel disease using an anti-CD81 antibody or a method for preventing, ameliorating, or treating refractory inflammatory bowel disease using an anti-CD81 antibody.

### Background Art

The intestine is the organ where nutrients and water, which are essential for vital activities of the living body, are digested and absorbed. Meanwhile, the intestine also has an immune defense function to get rid of foreign objects such as pathogens, and in this way it also serves as the organ that governs the maintenance of life by controlling the contrasting properties in a balanced manner. However, it is known that once the above functional balance is troubled, the state of dynamic equilibrium is disturbed, causing various intestinal diseases. Particularly, recently, an increase has been observed in the number of patients with inflammatory bowel disease (abbreviated as IBD), which is a disease that causes gastrointestinal abnormalities such as abdominal pain, diarrhea, and bloody stool with repeated relapse and remission. IBD is classified into ulcerative colitis (abbreviated as UC) and Crohn's disease (abbreviated as CD) based on its pathological condition.
UC is a disease that is mainly characterized by diffuse inflammation of the intestinal mucosa that is localized in the large intestine, and repeated inflammation leads to the development of colorectal cancer, which often ends up with the need for surgery. Even after surgery, there are problems of increased bowel movements, fecal incontinence, and development of pouchitis. With regard to UC, the following types of symptoms are known; first-attack-only type, relapse-remitting type, chronic-continuous type, and acute-fulminating type. While steroid is administered to severe cases of UC, more attentive care needs to be paid to the development of side effects (osteoporosis and susceptibility to infection) caused by steroid administration, etc., and thus it is advisable to keep in mind that unnecessarily prolonged steroid administration should be avoided.
CD is a lesion spanning from the small intestine to the large intestine, and it is a disease involving intense transmural inflammation mainly under discontinuous mucus, and repeated inflammation causes intestinal complications (stricture, fistulas, and abscess), leading to the need for surgery (Non Patent Literature 1).

The remission phase means not the curing of disease but the period in which the symptoms are subsided. For refractory diseases for which complete healing is hard to achieve like IBD, how long the period in which the symptoms are subsided can be maintained becomes important. With the current drug therapy, relapse of IBD is observed at a ratio of a certain number of patients not only after discontinuation of administration of drugs, but also during the administration of drugs, and in many cases it merely aims at the inhibition of the secretion and the action of inflammatory factors as a way of symptomatic treatment. Recently, there are cases in which anti-TNF-α antibody (Remicade or infliximab) is used for refractory IBD, in which obvious clinical symptoms that are attributable to the disease persist even after the implementation of proper treatment such as nutrition therapy and other drug therapies (such as a 5-aminosalicylic acid preparation). However, there is a report that relapse of disease is observed after single-dose administration of anti-TNF-α antibody, and even while the administration is continued, relapse is still observed in some patients (Non Patent Literature 2 and Non Patent Literature 3).

Meanwhile, CD81 is a 26 kDa surface molecule that is expressed in a wide variety of cells, and it acts to reduce the threshold for B cell activation by forming a complex with CD21, CD19, and Leu13 on B cells. On T cells, it assembles with CD4 and CD8 to transmit the stimulus information into the cell. Further, it is physiologically and functionally associated with various kinds of integrins to activate VLA-4 (α4β1 integrin) on B cells and LFA-1 (αLβ2 integrin) on thymocytes.
Pertaining to the anti-CD81 antibody, there is a report that single-dose administration of an anti-CD81 antibody inhibits 45 hours of the vascular permeability response that occurs in the model of passive cutaneous anaphylaxis, which is an allergic disease (Patent Literature 1). Also, there is a report that administration of an anti-CD81 antibody successfully inhibits 5 days of symptoms of single inflammatory lesions in the intestine that develop in the mouse colitis model, which is an animal model of inflammatory bowel disease (Patent Literature 2).
Further, there is also a report that symptoms of neuroparalysis with a single lesion that develop in the experimental autoimmune encephalomyelitis mouse, which is a model animal of multiple sclerosis, were successfully inhibited by a total of 10 times of every-other-day administration of Eat2, which is a clone of an anti-CD81 antibody, whereas the symptoms could not be inhibited by 2F7, which is another clone of an anti-CD81 antibody (Non Patent Literature 4).
As described above, a disease model in which amelioration is achieved with every-other-day administration of an anti-CD81 antibody has been reported, indicating that continuous administration of an anti-CD81 antibody is important for exertion of the efficacy.

### Citation List

### Patent Literature

Patent Literature 1: International Publication No. WO 1998/025647
Patent Literature 2: International Publication No. WO 2005/021792

### Non Patent Literature

Non Patent Literature 1: Inflamm. Bowel. Dis. 8, 244 to 250, 2002
Non Patent Literature 2: New England Journal of Medicine. 337, 1029 to 1035, 1997
Non Patent Literature 3: Journal of Clinical Gastroenterology. 41; 799 to 809, 2007
Non Patent Literature 4: Neurobiology of Disease 31, 413 to 421, 2008

### Summary of Invention

### Technical Problem

Creation of a drug capable of preventing, treating, or ameliorating the symptoms of refractory inflammatory bowel disease with repeated remission and relapse (recurrence) and a drug capable of maintaining remission of the symptoms of such disease is awaited. In other words, the current situation is that no progress has been made in the development of a drug for maintaining remission of inflammatory bowel disease for improvement of long-term prognosis. In light of the above, creation of a drug for improving long-term prognosis, particularly, a drug capable of reducing recurrence and surgery is demanded.
Accordingly, the present invention aims to provide a drug for maintaining remission of inflammatory bowel disease having not only a remission-inducing effect, but also a long-term remission-maintaining effect, and a method for maintaining remission of inflammatory bowel disease. The present invention further aims to provide a preventive, ameliorating, or therapeutic drug for refractory inflammatory bowel disease and a method for preventing, ameliorating, or treating refractory inflammatory bowel disease.

### Solution to Problem

The present inventors conducted an intensive research to achieve the aforementioned goals. As a result, they have found that an anti-CD81 antibody has not only a remission-inducing effect, but also a long-term remission-maintaining effect, and thus is useful as a drug for maintaining remission of inflammatory bowel disease, and also as a preventive, ameliorating, or therapeutic drug for refractory inflammatory bowel disease among other types of inflammatory bowel disease.
The present invention was completed based on the foregoing findings.

That is, the present invention relates to the following [1] to [32]:
[1]. a drug for maintaining remission of inflammatory bowel disease, comprising an anti-CD81 antibody as an active ingredient;
[2]. a preventive, ameliorating, or therapeutic drug for refractory inflammatory bowel disease, comprising an anti-CDB1 antibody as an active ingredient;
[3]. the preventive, ameliorating, or therapeutic drug according to the aforementioned [2], wherein the refractory inflammatory bowel disease refers to inflammatory bowel disease with repeated relapse or recurrence or inflammatory bowel disease with chronically persistent disease activity;
[4]. the preventive, ameliorating, or therapeutic drug according to the aforementioned [2] or [3], wherein the refractory inflammatory bowel disease is a steroid-dependent or steroid-resistant inflammatory disease;
[5]. the preventive, ameliorating, or therapeutic drug according to any one of the aforementioned [2] to [4], wherein the refractory inflammatory bowel disease refers to inflammatory bowel disease that is resistant or tolerant to usual treatment;
[6]. the preventive, ameliorating, or therapeutic drug according to the aforementioned [5], wherein the usual treatment refers to administration of an existing therapeutic drug;
[7]. the preventive, ameliorating, or therapeutic drug according to the aforementioned [6], wherein the existing therapeutic drug is a 5-aminosalicylic acid preparation, steroid, an immunosuppressant, a TNF inhibitor, or an integrin inhibitor;
[8]. the preventive, ameliorating, or therapeutic drug according to the aforementioned [7], wherein the existing therapeutic drug is sulfasalazine, an anti-TNF antibody, salazosulfapyridine, mesalazine, betamethasone, betamethasone sodium phosphate, betamethasone phosphate, prednisolone, azathioprine, tacrolimus, 6-mercaptopurine, cyclosporine, infliximab, adalimumab, certolizumab, pegol, or natalizumab;
[9]. the drug according to any one of the aforementioned [1] to [8], wherein the drug has a long-term remission-maintaining effect by single-dose administration;
[10]. the drug according to the aforementioned [9], wherein the remission-maintaining effect is an effect of maintaining a state of remission by inhibiting repetition of relapse or recurrence of intestinal inflammation;
[11]. the drug according to any one of the aforementioned [1] to [4], wherein the drug is administered to a patient with inflammatory bowel disease in whom remission cannot be maintained by single-dose or even by multiple-dose administration of an existing therapeutic drug;
[12]. the drug according to the aforementioned [11], wherein the existing drug is the existing drug according to the aforementioned [7] or [8];
[13]. the drug according to any one of the aforementioned [1] to [12], wherein the drug is administered once every 2 to 30 weeks during a, period of treatment;
[14]. the drug according to the aforementioned [13], wherein a dose per administration is 0.01 to 15 mg/kg;
[15]. the drug according to any one of the aforementioned [1] to [14], wherein the inflammatory bowel disease is ulcerative colitis;
[16]. the drug according to any one of the aforementioned [1] to [14], wherein the inflammatory bowel disease is Crohn's disease;
[17]. a method for maintaining remission of inflammatory bowel disease, comprising administering an anti-CD81 antibody to a subject in need thereof;
[18]. a method for preventing, ameliorating, or treating refractory inflammatory bowel disease, comprising administering an anti-CD81 antibody to a subject in need thereof;
[19]. the method according to the aforementioned [18], wherein the refractory inflammatory bowel disease refers to inflammatory bowel disease with repeated relapse or recurrence or inflammatory bowel disease with chronically persistent disease activity;
[20]. the method according to the aforementioned [18] or [19], wherein the refractory inflammatory bowel disease is a steroid-dependent or steroid-resistant inflammatory disease;
[21]. the method according to any one of the aforementioned [18] to [20], wherein the refractory inflammatory bowel disease is inflammatory bowel disease that is resistant or tolerant to usual treatment;
[22]. the method according to the aforementioned [21], wherein the usual treatment refers to administration of an existing therapeutic drug;
[23]. the method according to the aforementioned [22], wherein the existing therapeutic drug is a 5-aminosalicylic acid preparation, steroid, an immunosuppressant, a TNF inhibitor, or an integrin inhibitor;
[24]. the method according to the aforementioned [23], wherein the existing therapeutic drug is sulfasalazine, an anti-TNF antibody, salazosulfapyridine, mesalazine, betamethasone, betamethasone sodium phosphate, betamethasone phosphate, prednisolone, azathioprine, tacrolimus, 6-mercaptopurine, cyclosporine, infliximab, adalimumab, certolizumab, pegol, or natalizumab;
[25]. the method according to any one of the aforementioned [17] to [24], wherein a long-term remission-maintaining effect is exerted by single-dose administration;
[26]. the method according to the aforementioned [25], wherein the remission-maintaining effect is an effect of maintaining a state of remission by inhibiting repetition of relapse or recurrence of intestinal inflammation;
[27]. the method according to any one of the aforementioned [17] to [20], wherein an anti-CD81 antibody is administered to a patient with inflammatory bowel disease in whom remission cannot be maintained by single-dose administration or even by multiple-dose administration of an existing therapeutic drug;
[28]. the method according to the aforementioned [27], wherein the existing drug is the existing drug according to the aforementioned [23] or [24];
[29]. the method according to any one of the aforementioned [17] to [28], wherein the anti-CD81 antibody is administered once every 2 to 30 weeks during a period of treatment;
[30]. the method according to the aforementioned [29], wherein a dose per administration is 0.01 to 15 mg/kg;
[31]. the method according to any one of the aforementioned [17] to [30], wherein the inflammatory bowel disease is ulcerative colitis; and
[32]. the method according to any one of the aforementioned [17] to [30], wherein the inflammatory bowel disease is Crohn's disease.

### Advantageous Effects of Invention

The present invention revealed that an anti-CD81 antibody was capable of preventing, ameliorating, and treating refractory inflammatory bowel disease and an anti-CD81 antibody had a long-term remission-maintaining effect even by single-dose administration. In other words, the present invention revealed that an anti-CD81 antibody exerted a profound effect even in a patient with refractory inflammatory bowel disease (IBD) in whom single-dose administration or even multiple-dose administration of an existing therapeutic agent for inflammatory bowel disease has achieved only as far as a remission-inducing effect.
Accordingly, a drug containing an anti-CD81 antibody as the active ingredient exhibits not only a remission-inducing effect on inflammatory bowel disease, but also a long-term remission-maintaining effect by single-dose administration, and thus is utilizable as a drug for maintaining remission and as a preventive, ameliorating, or therapeutic drug for refractory inflammatory bowel disease. Further, an anti-CD81 antibody exhibits not only a remission-inducing effect, but also a long-term remission-maintaining effect on inflammatory bowel disease by single-dose administration, and thus single-dose administration of an anti-CD81 antibody is utilizable as a method for maintaining remission or a method for preventing, ameliorating, or treating refractory inflammatory bowel disease.

### Brief Description of Drawings

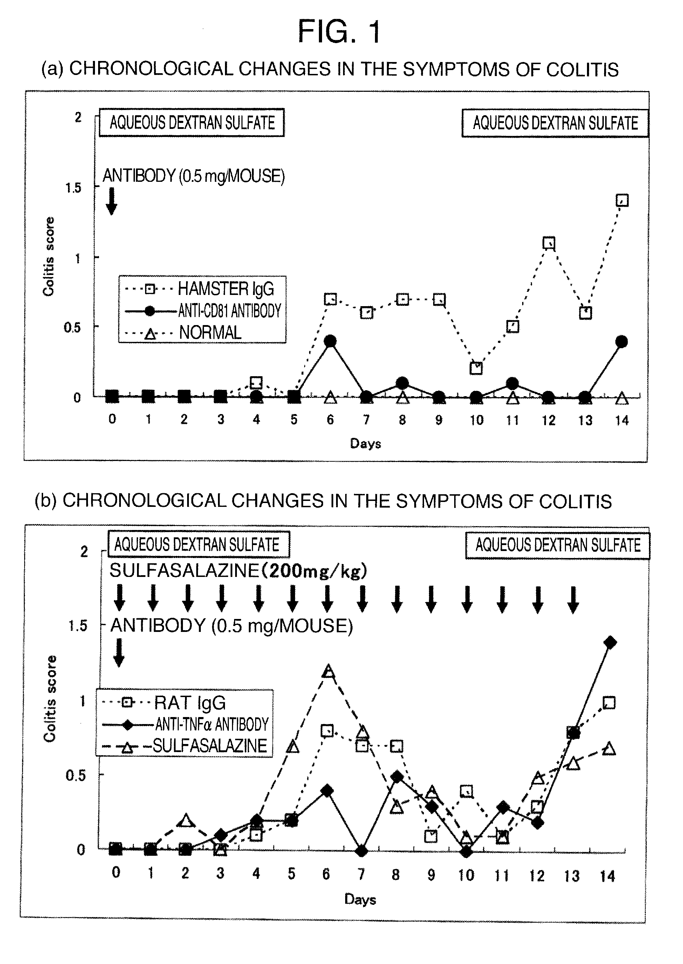

[Fig. 1] Fig. 1 (a) is a graph showing the chronological changes in the symptoms of colitis in the mouse model of relapse-remitting type ulcerative colitis by single-dose administration of either a hamster IgG antibody or an anti-CD81 antibody in the pharmacological test conducted in Example 1. Fig. 1(b) is a graph showing the chronological changes in the symptoms of colitis in the mouse model of relapse-remitting type ulcerative colitis by single-dose administration of either a rat IgG antibody or a rat anti-TNF-α antibody or by daily administration of sulfasalazine in the pharmacological test conducted in Example 1.

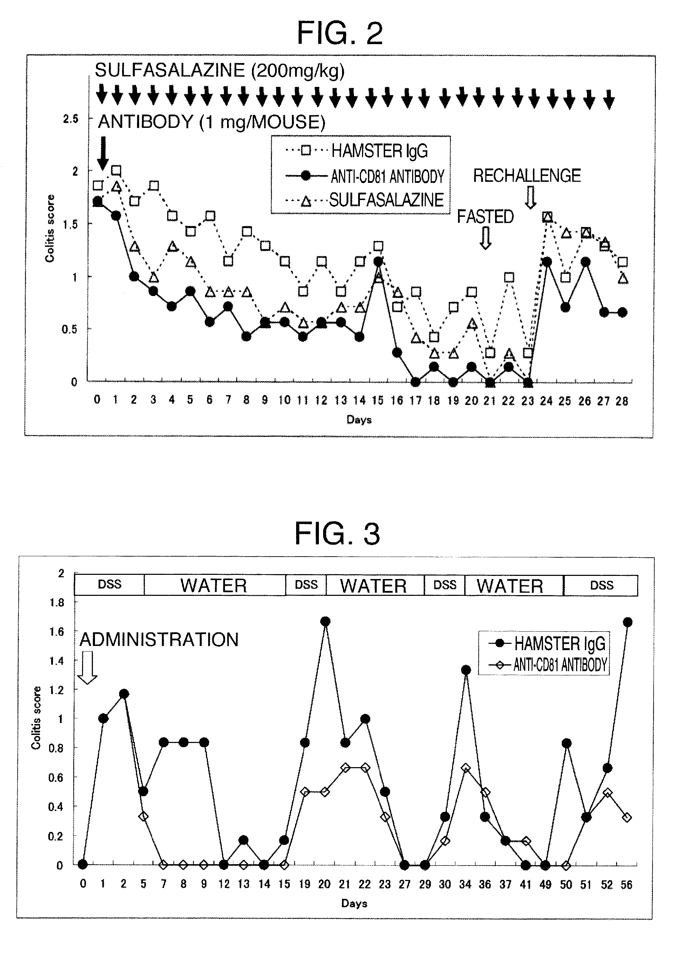

[Fig. 2] Fig. 2 is a graph showing the chronological changes in the symptoms of colitis in the mouse model of relapse-remitting type inflammatory bowel disease (Crohn's disease and ulcerative colitis) by single-dose administration of either a hamster IgG antibody or an anti-CD81 antibody or by daily administration of sulfasalazine in the pharmacological test conducted in Example 2.
[Fig. 3] Fig. 3 is a graph showing the chronological changes in the symptoms of colitis in the mouse model of relapse-remitting type ulcerative colitis by single-dose administration of either a hamster IgG antibody or an anti-CD8 antibody in the pharmacological test conducted in Example 3.

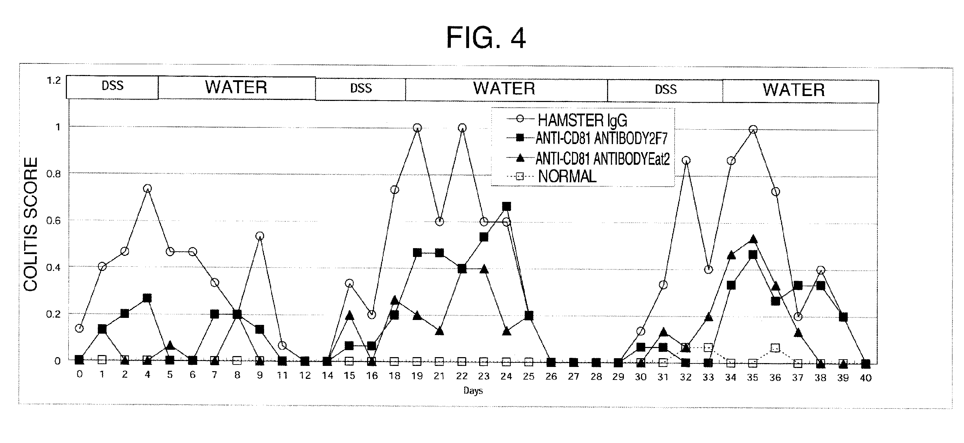

[Fig. 4] Fig. 4 is a graph showing the chronological changes in the symptoms of colitis in the mouse model of relapse-remitting type ulcerative colitis by single-dose administration of each of the hamster IgG antibody, the anti-CD81 antibody clone 2F7, and the anti-CD81 antibody clone Eat2 in the pharmacological test conducted in Example 4.

### Description of Embodiments

The following present invention will be described in detail.

### (1) Anti-CD81 antibody

The anti-CD81 antibody in the present invention may be an antibody that specifically recognizes CD81. Specifically, it may be an antibody that can specifically recognize the expression product (protein) of the CD81 gene (which may also be referred to as "CD81" in the present specification).
Here, CD81 encompasses not only a "protein" or "(poly)peptide" expressed by a specific amino acid sequence (SEQ ID NO: 2 (NP_004347)) indicating human CD81, but also its homologues substances (homologues and splice variants), mutants, derivatives, matured forms, amino acid-modified forms, and the like as long as they have equivalent biological function to the aforementioned protein or (poly)peptide. Here, examples of the homologue include a protein of other species of organism such as a mouse or a rat that corresponds to the human protein, which can be deductively identified based on the base sequence of a gene (SEQ ID NO: 1 (NM_004356)) published in http://www.ncbi.nlm.nih.gov. Also, the mutant encompasses a naturally occurring allelic mutant, a non-naturally-occurring mutant, and a mutant having a modified amino acid sequence by artificial deletion, substitution, addition, or insertion. Also, examples of the aforementioned mutant include one having at least 70% similarity, preferably 80% similarity, more preferably 95% similarity, and even more preferably 97% similarity to the non-mutated protein or (poly)peptide. Also, examples of the amino acid-modified form encompass a naturally-occurring amino acid-modified form and a non-naturally-occurring amino acid-modified form, and specific examples include one having a phosphorylated amino acid.

The aforementioned antibody used in the present invention includes a polyclonal antibody and a monoclonal antibody. Depending on the type of the constant domain of the heavy chain, an antibody is classified into five major classes; namely, IgA, IgD, IgE, IgG, or IgM. Some of them are further classified into subclasses or isotypes such as IgG1, IgG2, IgG3, and IgG4.
The antibody of the present invention is preferably a monoclonal antibody, and it encompasses a human antibody, a chimeric antibody, a humanized antibody, a single chain antibody, or a Fab fragment and a fragment produced by a Fab expression library, a low molecular weight antibody (including a fragment of an antibody), a polyspecific antibody, and further, a modified antibody. The antibody of the present invention also encompasses a part of the aforementioned antibody that has an antigen-binding property.

The phrase "specifically recognize" refers to binding to CD81.
As the anti-CD81 antibody used in the present invention, for example, a commercially available anti-CD81 antibody (the product of Pharmingen, Santa Cruz Biotechnology, Inc., BioLegend, Inc., SouthernBiotech, Ancell Corporation, MorphoSys AG, Chemicon, Abcam plc., ImmunoTech. Co., Ltd., R&D, and so on) may be used, while a polyclonal antibody or a monoclonal antibody produced by publicly known means may also be used.
As the anti-CD81 antibody used in the present invention, particularly, mammal-derived monoclonal and polyclonal antibodies are preferred. The monoclonal and polyclonal antibodies can be produced by a method publicly known to those skilled in the art.
Examples of the mammal-derived monoclonal and polyclonal antibodies include one produced in the blood of an animal, one produced by a hybridoma, one produced by a host transformed with an expression vector carrying the gene of the antibody by genetic engineering techniques, one obtained by mass-producing, in the CHO cell factory, the gene of the most suitable antibody that is screened from an enormous clone library consisting of a trillion molecules using phage display, or a human antibody directly obtained from a transgenic mouse producing a human antibody.

The protein that is used as a sensitizing antigen for acquisition of the anti-CD81 antibody of the present invention for the production of a polyclonal antibody is not limited by the animal species from which it is derived, which can be a human, a mouse, a rat, and the like. However, the protein is preferably selected in consideration of compatibility with the parent cell to be used for cell fusion, and it is generally preferably a mammal-derived protein, and particularly preferably a human-derived protein. For example, when CD81 is human CD81, a human CD81 protein, a cell expressing human CD81, a partial peptide of human CD81 can be used. Also, the protein may be a complete protein or a partial peptide of the protein. Examples of the partial peptide of the protein include an amino group (N) terminal fragment and a carboxy (C) terminal fragment of the protein. The anti-CD81 antibody in the present invention refers to an antibody that binds to the full-length protein or a fragment of the protein.
For example, a polyclonal antibody can be obtained as follows. That is, a small animal such as a rabbit is immunized with a naturally-occurring CD81 protein or a recombinant CD81 protein that is expressed in a microorganism such as E. coli as a fusion protein with GST, or a partial peptide thereof, and the serum of this animal is obtained. The serum thus obtained is purified by, for example, ammonium sulfate precipitation, a protein A column, a protein G column, DEAE ion-exchange chromatography, and an affinity column coupled with a CD81 protein or a synthetic peptide, whereby a polyclonal antibody is prepared.
The antigen can be prepared in accordance with, for example, a method using a baculovirus (for example, International Publication No. WO 98/46777). When the antigen has low antigenicity, it may be conjugated to an antigenic macromolecule such as albumin and then used for immunization.

As the method for producing a monoclonal antibody, an animal is immunized with a sensitizing antigen in accordance with a publicly known method. As a general method, a sensitizing antigen is administered to a mammal by an intraperitoneal or subcutaneous injection. Specifically, a sensitizing antigen is diluted to an appropriate dose and suspended in Phosphate-Buffered Saline (PBS), physiological saline, and the like, to which an appropriate amount of a common adjuvant, for example, the Freund's complete adjuvant, is added as desired, followed by emulsification. The resulting emulsion is administered to a mammal several times every four to 21 days. Also, when immunizing an animal with a sensitizing antigen, an appropriate carrier may be used.
Once a mammal is immunized as described above and an elevated serum level of the desired antibody is confirmed, immune cells are collected from the mammal and subjected to cell fusion. Examples of the particularly preferred immune cell include a spleen cell. As the counterpart parent cell to be fused with the aforementioned immune cell, a mammalian myeloma cell is used. As the myeloma cell, various kinds of publicly known cell lines, for example, P3U1 (P3-X63Ag8U1), P3 (P3x63Ag8.653) (J. Immunol. (1979) 123, 1548 to 1550), P3x63 Ag8U.1 (Current Topics in Microbiology and Immunology (1978) 81, 1 to 7), NS-1 (Kohler. G and Milstein, C., Eur. J. Immunol. (1976) 6, 511 to 519), MPC-11 (Margulies. D. H. et al., Cell (1976) 8, 405 to 415), SP2/0 (Shulman, M. et al., Nature (1978) 276, 269 to 270), FO (de St. Groth, S. F. et al., J. Immunol. Methods (1980) 35, 1 to 21), S194 (Trowbridge, I. S., J. Exp. Med. (1978) 148, 313 to 323), and R210 (Galfre, G. et al., Nature (1979) 277, 131 to 133) are preferably used.
The cell fusion of the aforementioned immune cell with the myeloma cell can basically be performed in accordance with a publicly known method, for example, the method of Kohler and Milstein, et al. (Kohler. G and Milstein, C., Methods Enzymol. (1981) 73, 3 to 46).

More specifically, the aforementioned cell fusion is performed, for example, in a common nutrient liquid culture medium in the presence of a cell fusion promoter. As the fusion promoter, for example, polyethylene glycol (PEG) and a hemagglutinating virus of Japan (HVJ) are used, and further, an auxiliary agent such as dimethyl sulfoxide may be added to increase the fusion efficiency as desired.
The ratio between the immune cell and the myeloma cell used can be arbitrarily set. For example, it is preferable to use one to 10 times the number of the immune cell than the myeloma cell. As the liquid culture medium used for the aforementioned cell fusion, for example, an RPMI 1640 medium, which is suitable for proliferation of the aforementioned myeloma cell line, a MEM medium, and in addition, common liquid culture media used for culturing these kinds of cells can be used, and further, a serum supplement such as fetal calf serum (FCS) can be used together.
In cell fusion, certain amounts of the aforementioned immune cells and myeloma cells are mixed well in the aforementioned liquid culture medium, to which a PEG solution (for example, an average molecular weight of approximately 1000 to 6000) that has been heated to approximately 37°C in advance, is added at a concentration of, normally, 30 to 60%(w/v), followed by mixing to form the desired hybridomas. Subsequently, an appropriate liquid culture medium is sequentially added, followed by centrifugation to remove a supernatant. This operation is repeated to remove a cell fusion agent and the like that are unfavorable for the growth of the hybridomas.

The hybridomas obtained as above are screened by culturing in a common selection medium, for example, a HAT medium (a liquid culture medium containing hypoxanthine, aminopterin, and thymidine). This culturing in a HAT medium is continued long enough (normally, several days to several weeks) for cells other than the desired hybridomas (unfused cells) to die out. Subsequently, common limiting dilution technique is applied to screen for a hybridoma producing the desired antibody, from which a single clone is produced.
In addition to obtaining the aforementioned hybridomas by immunizing an animal other than the human with an antigen, it is also possible to sensitize human lymphocytes, for example, EB virus-infected human lymphocytes, in vitro with a protein, a protein-expressing cell, or a lysed product of such a cell, and the sensitized lymphocytes are fused with immortalized human-derived myeloma cells, for example U266, to obtain a hybridoma producing a human antibody having the desired activity (for example, a cell migration-inhibiting activity).

The monoclonal antibody-producing hybridomas thus constructed can be subcultured in a common liquid culture medium, or can be stored for a prolonged period of time in liquid nitrogen. That is, the monoclonal antibody-producing hybridomas can be produced by, using the desired antigen or a cell expressing the desired antigen as the sensitizing antigen, immunizing a subject with this sensitizing antigen in accordance with a common immunization method; fusing the immune cell thus obtained with a publicly known parent cell by a common cell fusion method; and screening for a monoclonal antibody-producing cell (hybridoma) by a common screening method. In order to obtain the monoclonal antibody from the hybridoma, a method of culturing the hybridoma by a common method and obtaining the antibody as a culture supernatant, or a method of administering the hybridoma to a mammal compatible with the hybridoma, allowing the hybridoma to proliferate, and obtaining the antibody as ascites is adopted. The former method is suitable for obtaining high-purity antibodies, whereas the latter method is suitable for a large scale production of antibodies.

The human antibody refers to an antibody that is the expression product of the gene of the human-derived antibody. The human antibody can be obtained by, for example, administering an antigen to a transgenic animal made capable of producing a human-derived antibody through introduction of the human antibody gene locus. Examples of the transgenic animal include a mouse, and a method for producing a mouse capable of producing the human antibody is described in, for example, International Publication No. WO02/43478.
The monoclonal antibody according to the present invention also encompasses a monoclonal antibody composed of a heavy chain and/or light chain having an amino acid sequence resulting from deletion, substitution, or addition of one or several amino acids introduced in each of the amino acid sequences of the heavy chain and/or light chain composing the antibody. These partial modifications of amino acid (deletion, substitution, insertion, and addition) can be introduced in the amino acid sequence of the antibody of the present invention by partially modifying the base sequence encoding the amino acid sequence. Such partial modification of base sequence can be introduced by a routine method employing known site specific mutagenesis (Proc, Natl. Acsd. Sci. USA., 1984 Vol 81, 5662; Sambrook et al., Molecular Cloning A Laboratory Manual (1989) Second edition, Cold Spring Harbor Laboratory Press).
In the present invention, a gene recombinant antibody that has been artificially modified for the purpose of lowering heterologous antigenicity against humans, etc. such as a chimeric antibody and a humanized antibody can also be used. These modified antibodies can be produced using known methods.

A chimeric antibody is an immunoglobulin molecule characterized by binding of two or more moieties derived from heterologous animal species. Generally, the variable region (V region) of a chimeric antibody is derived from a mammalian antibody other than the human (such as a mouse monoclonal antibody), and the immunoglobulin constant region (C region) of a chimeric antibody is derived from a human immunoglobulin molecule. Preferably, a variable region having low antigenicity is selected, which is combined with a human constant region similarly having low antigenicity. Preferably, the resulting combination similarly has low antigenicity. The chimeric antibody encompasses monovalent immunoglobulin, bivalent immunoglobulin, and polyvalent immunoglobulin. The monovalent chimeric antibody is a dimer (HL) formed by a chimeric H chain bound to a chimeric L chain via a disulfide bridge. The bivalent antibody is a tetramer (H2L2) formed by two HL dimers bound together via at least one disulfide bridge.
The chimeric antibodies and the methods for producing the same are already described in the art (Morrison et al., Proc. Natl. Acad. Sci. USA 81: 6851 to 6855 (1984); Boulianne et al., Nature 312: 643 to 646 (1984); Liu et al., Proc. Natl. Acad. Sci. USA 84 : 3439 to 3443 (1987); Sun et al., Proc. Natl. Acad. Sci. USA, 84: 214 to 218 (1987); Better et al., Science 240: 1041 to 1043 (1988); and Harlow and Lane, ANTIBODIES: A LABORATORY MANUAL Cold Spring Harbor Laboratory(1988)).

The humanized antibody, which is also called a reshaped human antibody, is made by grafting the complementarity determining region (CDR) of an antibody of a mammal other than the human, for example a mouse antibody, into the complementarity determining region of a human antibody. General gene recombination technology for this is also known (see European Patent Application Publication No. EP 125023 and International Publication No. WO92/19759). As the method for producing a humanized antibody, a publicly known method can be used. For example, a DNA sequence designed to link the CDR of a mouse antibody and the framework region (FR) of a human antibody is synthesized by the PCR method from several oligonucleotides produced in such a way as to have sections overlapping with one another at the ends thereof. The DNA thus obtained is ligated to the DNA encoding the C region of a human antibody, and the resulting ligated DNA is inserted into an expression vector, which is then introduced into a host so that it will produce the antibody, whereby the humanized antibody is obtained (see European Patent Application Publication No. EP 239400 and International Publication No. WO92/19759). The framework region of a human antibody to be linked via CDR is selected so that the complementarity determining region forms a favorable antigen-binding site. If necessary, amino acids in the framework region of the variable region in the antibody may be substituted so that the complementarity determining region of a reshaped human antibody forms an appropriate antigen-biding site (Sato, K. et al., Cancer Res. (1993) 53, 851 to 856).
For a chimeric antibody and a humanized antibody, the human antibody C region is used. Examples of the human antibody C region include Cγ, and for example, Crγ1, Cγ2, Cγ3, or Cγ4 can be used. Also, in order to improve the stability of the antibody or the production of the antibody, the human antibody C region may be modified. A chimeric antibody is composed of the variable region of an antibody derived from a mammal other than the human and the C region derived from a human antibody, and a humanized antibody is composed of the complementarity determining region of an antibody derived from a mammal other than the human and the framework region and C region derived from a human antibody. Because these antibodies have reduced antigenicity in the human body, they are useful as the antibody to be used in the present invention.

The antibody used in the present invention may be a fragment of an antibody or a modified antibody as long as it is preferably used in the present invention. Examples of the fragment of an antibody include Fab, F(ab')2, Fv, or a single chain Fv (scFv) diabody in which Fv of the H and L chains is linked via an appropriate linker, a low molecular weight antibody, and a single chain antibody.
The low molecular weight antibody encompasses a fragment of an antibody, which is a partially-deleted whole antibody (for example, whole IgG), and no particularly limitation is imposed as long as it retains binding ability to the antigen (CD81 protein). The fragment of an antibody is not particularly limited as long as it is a part of the whole antibody, and it preferably contains the heavy chain variable region (VH) or/and the light chain variable region (VL).

The single chain antibody is also called "single chain Fv", namely a "sFv" antibody fragment. It has the VH and VL domains of an antibody, and these domains are present in a polypeptide single chain. The "Fv" fragment is the smallest fragment of an antibody, which contains complete antigen recognition and binding sites. Generally, an "Fv" fragment is a dimer (VH-VL dimer) in which a VH and a VL are strongly linked by non-covalent binding. The three complementarity determining regions (CDRs) of each of the variable regions interact with each other to form an antigen-binding site on the surface of the VH-VL dimer. Six CDRs confer the antigen binding site to an antibody. However, one variable region (or half of the Fv containing only three antigen-specific CDRs) alone is capable of recognizing and binding to an antigen, although its affinity is lower than the affinity of the entire binding site. An Fv polypeptide further contains a polypeptide linker between the VH and VL domains so that sFv forms the desired configuration for antigen binding. (Pluckthun, The Pharmacology of Monoclonal Antibodies, vol. 113, Rosenberg and Moore eds. Springer-Verlag, New York, pp. 269 to 315 (1994)).

As to the method for producing a low molecular weight antibody and a single chain antibody, for example, a fragment of an antibody is produced by treating an antibody with an enzyme such as papain and pepsin, or by constructing a gene encoding such a fragment of an antibody, introducing the resulting gene into an expression vector, and then expressing the gene in an appropriate host cell (for example, see Co, M. S. et al., J. Immunol. (1994) 152, 2968 to 2976, Better, M. & Horwitz, A. H. Methods in Enzymology (1989) 178, 476 to 496, Plueckthun, A. & Skerra, A. Methods in Enzymology (1989) 178, 497 to 515, Lamoyi, E., Methods in Enzymology (1986) 121, 652 to 663, Rousseaux, J. et al., Methods in Enzymology (1986) 121, 663 to 669, and Bird, R. E. et al., TIBTECH (1991) 9, 132 to 137).
ScFv is obtainable by linking the H chain V region and the L chain V region of an antibody. These regions are present in a single polypeptide chain. Generally, an Fv polypeptide further contains a polypeptide linker between VH and VL, whereby scFv can form a structure necessary for antigen binding (for general reviews of scFv, see Pluckthun "The Pharmacology of Monoclonal Antibodies" Vol. 113 (Rosenberg and Moore eds. (Springer Verlag, New York) pp. 269 to 315, 1994)). The linker is not particularly limited as long as it does not inhibit the expression of the antibody variable regions linked to both ends thereof
In such scFv, the H chain V region and the L chain V region are linked via a linker, preferably by a peptide linker (Huston, J. S. et al., Proc. Natl. Acad. Sci. U.S.A. (1988) 85, 5879 to 5883). The H chain V region and the L chain V region in scFv may be derived from any of the substances listed as an antibody above. As the peptide linker linking the V regions, for example, any single chain peptide consisting of 12 to 19 amino acid residues is used.
DNA encoding scFv is obtained as follows; using DNA encoding the H chain or the H chain V region of an antibody and DNA encoding the L chain or the L chain V region of an antibody as described above as a template, the segment of DNA encoding the desired amino acid sequences within the sequences of the above parts of an antibody is amplified by PCR using a pair of primers that define both ends of the DNA. Subsequently, amplification is performed by combining DNA encoding the peptide linker moiety and a pair of primers that define so that each of the both ends of the DNA is linked to the H chain or the L chain, respectively. Further, once the DNA encoding scFv is produced, an expression vector carrying the DNA and a host transformed with the expression vector can be obtained in accordance with a routine method, and scFv can be obtained in accordance with a routine method by using the host thus obtained.

A diabody refers to a bivalent antibody fragment constructed by gene fusion (for example, Holliger, P. et al., Proc. Natl. Acad. Sci. USA 90: 6444 to 6448 (1993), EP Patent Application Publication No. 404097, and International Publication No. WO93/11161). A diabody is a dimer composed of two polypeptide chains, and normally in each polypeptide chain, VL and VH are linked by a linker that is short enough to prevent them from binding to each other within the same chain, for example, a linker of approximately five residues. The VL and VH encoded on the same polypeptide chain form a dimer since the linker between them is too short to allow them to form a single chain variable region fragment. Consequently, a diabody possesses two antigen-binding sites.
Also, sc(Fv)2 is a low molecular weight antibody in which two VHs and two VLs are linked together by a linker and the like into a single chain (Hudson et al., J Immunol. Methods 1999; 231: 177 to 189). The sc(Fv)2 can be produced by, for example, linking scFvs by a linker.

In the present invention, as the linker linking the variable regions of an antibody, any peptide linker that can be introduced by genetic engineering or a synthetic compound linker, i.e., a linker disclosed in (for example, see Protein Engineering, 9 (3), 299 to 305, 1996), and the like can be used. When a peptide linker is used, the length of the linker is not particularly limited, and it can be appropriately selected by those skilled in the art according to the purpose; however, normally, it consists of one to 100 amino acids, preferably three to 50 amino acids, more preferably five to 30 amino acids, and particularly preferably 12 to 18 amino acids (for example, 15 amino acids). The synthetic compound linker (chemical cross-linking agent) is a cross-linking agent normally used for cross-linking of peptides, and examples thereof include N-hydroxysuccinimide (NHS), disuccinimidyl suberate (DSS), bis(sulfosuccinimidyl)suberate (BS3), dithiobis(succinimidyl propionate) (DSP), dithiobis(sulfosuccinimidyl propionate) (DTSSP), ethylene glycol bis(succinimidyl succinate) (EGS) ethylene glycol bis(sulfosuccinimidyl succinate) (sulfo-EGS), disuccinimidyl tartrate (DST), disulfosuccinimidyl tartrate (sulfo-DST), bis[2-(succinimidooxycarbonyloxy)ethyl]sulphone (BSOCOES), and bis[2-(sulfosuccinimidoaxycarbanyloxy)ethyl]sulphane (sulfo-BSOCOES), and these cross-linking agents are commercially available.

These fragments of an antibody can be produced by acquiring the gene thereof and expressing it in a host in a similar manner to the above. The "antibody" as referred to in the claims of the present application encompasses these fragments of an antibody.
As a modified antibody, an antibody conjugated with various kinds of molecules such as polyethylene glycol (PEG) can also be used. The "antibody" as referred to in the claims of the present application also encompasses these modified antibodies. Theses modified antibodies can be obtained by subjecting the antibody thus obtained to chemical modification. The method of antibody modification has already been established in the art.

The antibody produced and expressed as described above can be isolated from the inside and outside of the cell as well as from the host, and purified to homogeneity. The antibody used in the present invention can be isolated and purified by affinity chromatography. Examples of the column used for affinity chromatography include a protein A column and a protein G column. Examples of the carrier used in the protein A column include Hyper D, POROS, and Sepharose F.F. Besides the above, common methods of isolation and purification of protein may be used, and no limitation is imposed on the method.
For example, the antibody used in the present invention can be isolated and purified by appropriately selecting and combining chromatography other than the aforementioned affinity chromatography, a filter, ultrafiltration, salting-out, dialysis, and the like. Examples of the chromatography include ion exchange chromatography, hydrophobic chromatography, and gel filtration. The above chromatography can be applied to High performance liquid chromatography (HPLC). Further, reverse-phase HPLC may also be used.

### (2) Inflammatory bowel disease

Inflammatory bowel disease is a disease that causes intestinal inflammation with repeated severe abdominal pain and diarrhea. Inflammatory bowel disease is classified into Ulcerative Colitis (abbreviated as UC) and Crohn's disease (abbreviated as CD) according to its pathological condition, and in both cases the disease persists for a long time and often involves repeated remission and relapse.

At present, regarding the indication of a therapeutic pharmaceutical product for inflammatory bowel disease, for example, azathioprine, which is an immunosuppressant, is approved to be used for "remission induction and remission maintenance of steroid-dependent Crohn's disease and remission maintenance of steroid-dependent ulcerative colitis" in Japan, and tacrolimus, which is an immunosuppressant, is approved to be used for "refractory (steroid-resistant and steroid-dependent) active ulcerative colitis (limited to moderate to severe UC)" in Japan. Also, for Crohn's disease, it is specified that infliximab, which is an anti-TNF antibody, "should be administered when obvious clinical symptoms that are attributable to the disease persist even after the implementation of proper treatment such as nutrition therapy and other drug therapies (such as a 5-aminosalicylic acid preparation)" in Japan. Further, in the U.S., infliximab is approved to be used for Crohn's disease "for inhibition, remission induction, and remission maintenance of signs and symptoms in adults with moderate to severe Crohn's disease that is resistant to usual treatment and in patients with juvenile active Crohn's disease, and for reduction in the number of enterocutaneous fistula and rectovaginal fistula and for maintenance of fistula closure in patients with adult Crohn's disease with fistula", and for ulcerative colitis, "for inhibition of signs and symptoms as well as induction and maintenance of clinical remission and mucosal healing in patients with moderate to severe active ulcerative colitis that is resistant to usual treatment."

As described above, in the treatment of inflammatory bowel disease, the usage of a therapeutic pharmaceutical product is approved for remission induction and remission maintenance, and the states of disease are distinguished in terms of the concept of severity of disease (mild to severe), the presence or absence of resistance to existing drugs, intractability, and the like. Here, remission induction refers to inducing remission in the active lesions involving bloody stool, ulcer and erosion in the intestinal tract, reduced QOL, and the like. Also, remission maintenance refers to inhibiting the repetition of relapse/recurrence of the active lesions and maintaining the remission state for as long as possible. Here, the term "remission" refers to an improvement in an activity index, where the activity index is obtained by expressing the disease activity such as diarrhea, melena, abdominal pain, pyrexia, body weight reduction, and anal pain, all of which are the symptoms of inflammatory bowel disease, in terms of Crohn's disease activity index (CDAI), IOIBD assessment score, Dutch AI, ulcerative colitis activity index, and the like.
The term "refractory (intractability)" refers to a state in which repeated relapses occur and clinical symptoms attributable to the disease persist, i.e., the disease activity chronically persists, even with proper implementation of usual treatment such as nutrition therapy and other drug therapies. The phrase "the disease activity chronically persists" refers to a state in which the symptoms persists for six months or more after the initiation of treatment, and this encompasses a condition (disease) that is resistant or tolerant to usual treatment such as a steroid-dependent or steroid-resistant condition or disease.
Examples of the existing drug used for usual treatment include a 5-aminosalicylic acid preparation (for example, salazosulfapyridine, sulfasalazine, and mesalazine), steroid (for example, betamethasone, betamethasone sodium phosphate, betamethasone phosphate, and prednisolone), an immunosuppressant (for example, azathioprine, tacrolimus, 6-mercaptopurine, and cyclosporine), a TNF inhibitor (for example, infliximab, adalimumab, certolizumab, pegol), and an integrin inhibitor (for example, natalizumab).

The present invention provides a drug containing an anti-CD81 antibody as the active ingredient that has a long-term remission-maintaining effect and is effective for ameliorating or treating relapse-remitting type inflammatory bowel disease (IBD) among other types of IBD by single-dose administration. In the present invention, the term "relapse" refers to redevelopment of symptoms after a certain period of time after the subsidence of the symptoms of inflammatory bowel disease, and the term "recurrence" also has the same meaning.
Patent Literature 2 (International Publication No. WO/2005/021792) has introduced a novel finding that CD81 is highly expressed in IBD-pathogenic cells, and an anti-CD81 antibody exerts a therapeutic effect in the mouse model of first-attack-only type colitis. However, International Publication No. WO/2005/021792 does not reveal that an anti-CD81 antibody is also effective for prevention, amelioration, and treatment of relapse-remitting type and chronic continuous type refractory inflammatory bowel disease with repeated relapse (recurrence) and remission. The present inventors produced a novel animal model presenting with inflammatory bowel disease accompanying relapse-remitting type symptoms and newly found that only single-dose administration of an anti-CD81 antibody to this animal model successfully inhibited persistence and relapse (recurrence) of intestinal inflammation. The present invention is based on a novel finding that an anti-CD81 antibody is also effective for prevention, amelioration, and treatment of relapse-remitting type (symptoms of repeated relapse (recurrence) and remission) and chronic continuous type refractory inflammatory bowel disease.
Accordingly, the drug for maintaining remission of IBD or the preventive, ameliorating, or therapeutic drug for refractory IBD provided by the present invention contains an anti-CD81 antibody, which has the remission-maintaining effect that the existing therapeutic agents do not have. In other words, in a patient in whom symptoms of repeated relapse (recurrence) and remission cannot be prevented, ameliorated, or treated by single-dose or even by multiple-dose administration of existing therapeutic agents, such symptoms can be prevented, ameliorated, or treated even by single-dose administration.

The drug containing an anti-CD8 antibody as the active ingredient can be administered either orally or parenterally; however, it is preferably administered parenterally. Specific examples of the dosage form include an injection form, a nasal administration form, a pulmonary administration form, and a transdermal administration form. As an example of the injection form, the drug can be administered systemically or locally by, for example, intravenous injection, intramuscular injection, intraperitoneal injection, and subcutaneous injection. The dosage varies depending on the kind of active ingredient, the administration route, the age, body weight, and symptoms of the subj ect of administration or the patient, and the like, and therefore cannot be flatly defined; however, normally, as a dose per administration, it is administered at 0.01 to 15 mg/kg, preferably at 1 to 15 mg/kg, more preferably at 3 to 5 mg/kg, or preferably at 0.01 to 5 mg/kg, more preferably at 0.05 to 5 mg/kg. As to the administration interval, no subsequent administration may be given as long as the effect continues. For example, during the treatment period, it is administered once every 2 to 30 weeks, 2 to 15 weeks, 2 to 13 weeks, 2 to 10 weeks, or 2 to 8 weeks, or it may be administered at a frequency of once every several weeks to several months or once every year to several years. In other words, as a dose per administration, an anti-CD81 antibody is normally administered at 0.01 to 15 mg/kg, preferably at 1 to 15 mg/kg, more preferably at 3 to 5 mg/kg, or preferably at 0.01 to 5 mg/kg, more preferably at 0.05 to 5 mg/kg, thereby exerting a remission-maintaining effect for as long as 2 to 13 weeks, 2 to 10 weeks, or 2 to 8 weeks. Also, when sufficient efficacy cannot be obtained or the efficacy is diminished, setting the above dosage at one cycle, multiple cycles can be administered (for example, 2 to 5 times), whereby the efficacy is achieved and then maintained.

The drug containing an anti-CD81 antibody as the active ingredient per se is administered as a drug prepared by a publicly known drug formulation method. For example, it can be used in the form of an injection of a sterile solution or a suspension prepared with water or a pharmaceutically acceptable liquid other than water. Also, for example, the drug can be prepared by combining the antibody with a pharmaceutically acceptable carrier or medium, specifically, sterilized water, physiological saline, an emulsifier, a suspending agent, a surfactant, a stabilizer, a vehicle, a preservative, and the like and mixing them in a unit dosage form required for generally approved practice of drug manufacture. The amount of the active ingredient in these preparations is adjusted such that an appropriate amount in the prescribed range is obtained.
A sterile composition for injection can be formulated in accordance with common practice of drug manufacture using a vehicle such as distilled water for injection. Examples of an aqueous solution for injection include physiological saline and an isotonic solution containing glucose and other adjunct agents such as D-sorbitol, D-mannose, D-mannitol, and sodium chloride, and an appropriate solubilizing agent such as alcohol, specifically, ethanol, polyalcohol such as propylene glycol and polyethylene glycol, and a nonionic surfactant such as polysorbate 80TM and HCO-50 can be used together.
Examples of an oily solution include sesame oil and soybean oil, and these oils may be used with benzyl benzoate and benzyl alcohol as a solubilizing agent. Also, a buffer such as a phosphate buffer and a sodium acetate buffer, a soothing agent such as procaine hydrochloride, a stabilizer such as benzyl alcohol, phenol, and an antioxidant may be added. The injection solution thus prepared is normally placed in an appropriate ampoule.

### Examples

Next, the present invention will be explained further specifically with reference to Examples. However, the present invention is not limited in any way by these Examples.

### Example 1

### Relapse-inhibitory effect of single-dose administration of an anti-CD81 antibody in the mouse model of colitis induced by an aqueous solution of dextran sulfate (DSS)

An aqueous solution of dextran sulfate (DSS) was administered to a mouse to induce relapse-remitting type ulcerative colitis. To this mouse model of relapse-remitting type ulcerative colitis, an anti-CD81 antibody was administered once and a relapse (recurrence)-inhibitory effect of single-dose administration of an anti-CD81 antibody on ulcerative colitis was examined by comparing with single-dose administration of an anti-TNF-α antibody and multiple-dose administration of sulfasalazine, both of which are the existing therapeutic agents.

### 1. Method

### (1) Preparation of DSS

Dextran sulfate (the product of TdB consultancy AB, an average molecular weight of 47,000) was dissolved in drinking water to prepare a 1% aqueous solution.

### (2) Preparation of an administration solution

As the test drug to be studied, a hamster anti-CD81 1 antibody, a rat anti-TNF-α antibody, and sulfasalazine were used, and as the pathological condition control test drug, rat IgG and hamster IgG were used.
Phosphate buffer was added to the concentrated original solutions of the hamster anti-CD81 antibody (Clone: 2F7, the product of SouthernBiotech, a monoclonal antibody for mouse CD81) and rat IgG and to the original solutions of the rat anti-TNF-α antibody and hamster IgG, whereby 0.25 mg/ml administration solutions were prepared. As to sulfasalazine (the product of Sigma), it was suspended in a 0.5% aqueous solution of methyl cellulose (the product of Nacalai Tesque, Inc.) in distilled water for injection (the product of Otsuka Pharmaceutical Co., Ltd.) at 20 mg/ml, whereby an administration solution was prepared.

### (3) Grouping, DSS ingestion, and drug administration

Four-week-old mice (BALB/cAnNCrj, male, the product of Charles River Laboratories Japan, Inc.) were purchased, and the mice in which no abnormality was found after six days of quarantine rearing were measured for body weight and then divided into six groups, where one group contained 10 mice. For five days after grouping, five groups of mice were fed 1% DSS ad libitum (first DSS ingestion), while one group of mice were fed normal drinking water as a normal mouse group. After five days of DSS ingestion, all six groups of mice were fed normal drinking water for five days. After ingestion of normal drinking water, five groups of mice were fed 1% DSS ad libitum (second DSS ingestion), while one group of mice were fed normal drinking water as a normal mouse group, and the test was completed. On the day of grouping, hamster IgG rat IgG, the anti-CD81 antibody, and the anti-TNF antibody were intraperitoneally administered at 0.5 mg/mouse once. From the day of grouping to the day before completion of the test, the sulfasalazine administration solution was orally administered once a day at 200 mg/kg.

### (4) Colitis symptom score assessment

From the day of grouping to the day of completion of the test, the symptoms of colitis were observed. The symptoms of colitis were assessed by score according to the condition of stool (normal stool (score: 0), loose stool (score: 1), and diarrhea (score: 2)).

### (5) Ulcer score assessment

On the day of completion of the experiment, 0.1 ml of a 2.5% brilliant blue 6B solution was administered to all groups of mice via tail vein. Ten minutes after the intravenous injection, the mice were euthanized by carbon dioxide gas, followed by blood removal by heart puncture. Then, a segment of the large intestine from 2 cm to 4 cm above the anus was taken out and thoroughly washed with physiological saline, which was then sandwiched between filter paper, fixed for 30 minutes in 75% ethanol, and immersed in physiological saline containing 5% hydrogen peroxide for 10 minutes. Subsequently, the large intestine was thoroughly washed with an ethanol solution and the size of ulcer was measured. The mouse with ulcer of 1 mm² or larger was assessed as ulcer score "1", whereas the mouse without ulcer of 1 mm² or larger was assessed as ulcer score "0."

### 2. Results

### (1) Chronological changes in the symptoms of colitis by colitis symptom score assessment

The chronological changes in the symptoms of colitis by colitis symptom score assessment in the mice that received single-dose administration of either one of hamster IgG and the hamster anti-CD81 antibody and in the mice that ingested normal drinking water (normal group) were shown in Fig. 1(a). Also, the chronological changes in the symptoms of colitis by colitis symptom score assessment in the mice that received single-dose administration of either one of rat IgG and the rat anti-TNF-α antibody and in the mice that received daily administration of sulfasalazine were shown in Fig. 1(b), In Fig. 1(a) and Fig. 1(b), Day 0 corresponds to the day of grouping, Days 0 to 4 correspond to the first DSS ingestion period, Days 5 to 9 correspond to the normal drinking water ingestion period, and Days 10 to 14 correspond to the second DSS ingestion period.
As is apparent from the hamster IgG administration group in Fig. 1(a) and the rat IgG administration group in Fig. 1(b), the first peak of the symptoms of colitis (first episode of colitis) after the first DSS ingestion was observed from Day 5 to Day 9 in Fig. 1(a) and Fig. 1(b), and the second symptoms of colitis at relapse (relapse colitis) were observed from Day 10 to Day 14 in Fig. 1(a) and Fig. 1(b). Also, compared to the inhibitory effect of daily administration of sulfasalazine and single-dose administration of the rat anti-TNF-α antibody on the first episode of colitis and on relapse colitis, single-dose administration of the hamster anti-CD81 antibody exhibited a stronger inhibitory effect on the first episode of colitis and on relapse colitis.

### (2) Colitis symptom score assessment and ulcer score assessment

Based on the results of the chronological changes in the symptoms of colitis in Fig. 1(a) and Fig. 1(b), an average colitis score of each of the mice from Day 5 to Day 9 was used as the first episode colitis score, and an average colitis score of each of the mice from Day 10 to Day 14 was used as the relapse colitis score. Further, a two group comparison was performed between each group and the pathological condition control group (hamster IgG administration group) with respect to the colitis score (first episode colitis score and relapse colitis score) and the ulcer score by the Wilcoxon test. The results thus obtained were shown in Tables 1 and 2.

[Table 1]

**Table 1: Results of the colitis score**

| Group | First episode colitis | score | Relapse colitis | score |
|---|---|---|---|---|
| | Score (SEM) | Significant difference | Score (SEM) | Significant difference |
| Pathological condition control (Hamster IgG) | 0.54 (0.14) | - | 0.76 (0.22) | - |
| Pathological condition control (Rat IgG) | 0.50 (0.15) | - | 0.52 (0.17) | - |
| Anti-TNF-α antibody administration | 0.28 (0.09) | No | 0.54 (0.25) | No |
| Anti-CD81 antibody administration | 0.10 (0.08) | * | 0.10 (0.08) | ** |
| Sulfasalazine administration | 0.68 (0.16) | No | 0.40 (0.13) | No |
| Normal | 0.00 (0.00) | ** | 0.00 (0.00) | ** |

| | | | | |
|---|---|---|---|---|
| *;0.01<p<0.05,**;p<0.01 | | | | |

[Table 2]

**Table 2: Results of the ulcer score**

| Group | Ulcer score | |
|---|---|---|
| | Score | Significant difference |
| Pathological condition control (Hamster IgG) | 0.4 | - |
| Pathological condition control (Rat IgG) | 0.3 | - |
| Anti-TNF-α antibody administration | 0.3 | No |
| Anti-CD81 antibody administration | 0.0 | * |
| Sulfasalazine administration | 0.1 | No |
| Normal | 0.0 | * |

| | | |
|---|---|---|
| *;0.01 <p<0.05, **;p<0.01 | | |

As is apparent from the results of Tables 1 and 2, compared to single-dose administration of the anti-TNF-α antibody and daily administration of sulfasalazine, both of which are the existing therapeutic drugs, single-dose administration of the anti-CD81 antibody could significantly and potently inhibit the symptoms of the first episode of colitis and relapse colitis, and after the first episode of colitis and relapse colitis, potently inhibit large intestinal ulcer. Accordingly, it was revealed that single-dose administration of the anti-CD81 antibody exhibited a long-term remission-maintaining effect on relapse-remitting type inflammatory bowel disease such as ulcerative colitis.

### Example 2

### Long-term remission effect of an anti-CD81 antibody in the mouse model of colitis induced by 2,4,6-trinitrobenzenesulfonic acid (TNBS)

The anti-CD81 antibody was administered once to the mouse model of relapse-remitting type inflammatory bowel disease (Crohn's disease and ulcerative colitis), which is obtained by administering TNBS to a mouse, and the relapse (recurrence)-inhibitory effect of single-dose administration of the anti-CD81 antibody on inflammatory bowel disease was examined by comparing with multiple-dose administration of sulfasalazine, which is the existing therapeutic drug.

### 1. Method

### (1) Preparation of TNP-OVA

Into 25 ml of distilled water for injection, 0.5 g of ovalbumin (OVA: Sigma) and 0.5 g of K₂CO₃ (Nacalai Tesque, Inc.) were dissolved (OVA solution). Into 25 ml of 0.1 M K₂CO₃, 0.5 g of 2,4,6-trinitrobenzenesulfonic acid (TNBS: Nacalai Tesque, Inc.) was dissolved (TNBS solution). The OVA solution and the TNBS solution were mixed and stirred overnight at room temperature. Then, the solution after stirring was dialyzed against 0.01 M NaHCO₃ (Nacalai Tesque, Inc.) through a dialysis membrane (Spectra/Por Membrane MWCO: 10,000, Spectrum Medical Industries, Inc.). The solution after dialysis was subjected to protein quantitation using the BCA protein assay reagent (Pierce).

### (2) Grouping, sensitization, challenge, relapse, and drug administration

Five-week-old mice (SJL/JorlIcoCrj, male, Charles River Laboratories Japan, Inc.) were administered (sensitized) with a 1 : 1 emulsion of complete Freund's adjuvant (CFA: Difco Laboratories) and 2 mg/ml TNP-OVA by subcutaneous injection in the back at 0.1 ml/head. Seven days after sensitization, a 10 mg/ml solution of TNBS in 50% ethanol was infused into the intestine under ether anesthesia (infused at 0.2 ml/head through a sonde that was inserted up to 3 cm from the anus: challenge). Six days after the challenge, the mice were divided into four groups, where one group contained seven mice, according to the body weight and symptom score. Twenty one days after the grouping, the mice were fasted for one day, and 23 days after the grouping, a 10 mg/ml solution of TNBS in 50% ethanol was infused into the intestine again (infused at 0.2 ml/head through a sonde that was inserted up to 3 cm from the anus: challenge). The test was completed 28 days after the grouping.
Four groups were each assigned as a 1 mg (0.2 ml)/head anti-CD81 antibody solution (Clone: 2F7, the product of SouthernBiotech) administration group, a 0.1 mg (0.2 ml)/head anti-CD81 antibody solution (Clone: 2F7, the product of SouthernBiotech) administration group, a 0.2 ml/head control antibody-administered pathological condition control group, and a 200 mg/kg sulfasalazine (SSZ: Sigma) administration group. As the control antibody, hamster IgG was used.
As to the drug administration, the respective antibodies were intraperitoneally administered to the antibody administration group and to the pathological condition control group once on the day of grouping, while SSZ was orally administered every day, once daily, from the day of grouping. The test was completed 28 days after the grouping. The SSZ solution was suspended in a 0.5% solution of methyl cellulose (Nacalai Tesque, Inc.).

### (3) Symptom score assessment

The symptoms of colitis were observed from Day 15 to Day 28 after the grouping (after single-dose administration of the anti-CD81 antibody) to examine the persistence of the effect. The symptoms of intestinal inflammation were assessed by score according to the condition of stool (normal stool (score: 0), loose stool (score: 1), and diarrhea (score: 2)). The dead mice were excluded from the results. For statistical processing, a two group comparison was performed between the pathological condition control group and the anti-CD81 antibody administration group or the SSZ administration group by the Wilcoxon rank sum test.

### 2. Results

An average colitis symptom score of each group from Day 15 to Day 28 after the grouping (after single-dose administration of the anti-CD81 antibody) and the standard error (SEM) were shown in Table 3. Also, in Fig. 2, the chronological changes in the symptoms of colitis in the hamster IgG single-dose administration group, the 1 mg (0.2 ml)/head anti-CD81 antibody single-dose administration group, and the sulfasalazine daily administration group were shown.

[Table 3]

**Table 3 : Results of the colitis score assessment**

| Group | Symptoms of colitis (SEM) |
|---|---|
| Pathological condition control | 1.16(0.34) |
| Anti-CD81 antibody administration (Single-dose administration; 1 mg/head) | 0.61 (0.16)* |
| Anti-CD81 antibody administration (Single-dose administration; 0.1 mg/head) | 0.84 (0.12) |
| SSZ administration | 0 87 (0.09) |
| (Daily multiple-dose administration; 200 mg/kg) | |

| | |
|---|---|
| *;0.01<p<0.05,**;p<0.01 | |

From the results of Table 3, it was revealed that the ameliorating effect on the symptoms of intestinal inflammation in the TNBS-induced colitis mice persisted from Day 15 to Day 28 after single-dose administration of the anti-CD81 antibody (1 mg/head). Also, from the results of Fig. 2, it was revealed that the anti-CD81 antibody exerted a long-term remission-maintaining effect in the inflammatory bowel disease model by single-dose administration.

### Example 3

### Relapse-inhibitory effect of single-dose administration of an anti-CD81 antibody in the mouse model of colitis induced by an aqueous solution of dextran sulfate (DSS)

The anti-CD81 antibody was administered once to the mouse model of relapse-remitting type ulcerative colitis, which is obtained by administering an aqueous solution of dextran sulfate (DSS) to a mouse, and the relapse-inhibitory effect of the anti-CD81 antibody on relapse-remitting type ulcerative colitis that was induced for a longer period of time was examined.

### 1. Method

### (1) Reparation of DSS

Dextran sulfate (the product of Wako Pure Chemical Industries, Ltd., an average molecular weight of 5,000) was dissolved in drinking water to prepare a 2% aqueous solution.

### (2) Preparation of an administration solution

As the test drug to be studied, a hamster anti-CD81 antibody was used, and as the pathological condition control test drug, hamster IgG was used. The concentrated original solution of the hamster anti-CD81 antibody (Clone: 2F7, the product of SouthernBiotech) was used as the administration solution, while phosphate buffer was added to the original solution of hamster IgG to prepare a 0.25 mg/ml administration solution.

### (3) Grouping, DSS ingestion, and drug administration

Four-week-old mice (BALB/cAnNCrlCrlj, male, the product of Charles River Laboratories Japan, Inc.) were purchased, and the mice were reared for quarantine for seven days, and then reared conventionally for seven days. Thereafter, the mice were measured for body weight and divided into two groups, where one group contained six mice. On the day of grouping, hamster IgG or the anti-CD81 antibody was intraperitoneally administered once at 0.5 mg/mouse. All mice were fed 2% DSS ad libitum (first DSS ingestion) for five days after the grouping, and then normal drinking water for the next 10 days. Subsequently, all mice were fed 2% DSS ad libitum (second DSS ingestion) for five days, and then normal drinking water for the next nine days. Subsequently, all mice were fed 2% DSS ad libitum (third DSS ingestion) for five days, and then normal drinking water for the next 16 days. Subsequently, all mice were fed 2% DSS ad libitum (forth DSS ingestion) for seven days, and the test was completed.

### (4) Colitis symptom score assessment

From the day of grouping to the day of completion of the test, the symptoms of colitis were observed. The symptoms of colitis were assessed by score according to the condition of stool (normal stool (score: 0), loose stool (score: 1), and diarrhea (score: 2)).

### 2. Results

The chronological changes in the symptoms of colitis by colitis symptom score assessment in the mice that received single-dose administration of either one of hamster IgG or the hamster anti-CD81 antibody were shown in Fig. 3. In Fig. 3, Day 0 corresponds to the day of grouping, Day 0 to Day 5 correspond to the first DSS ingestion period, Day 15 to Day 20 correspond to the second DSS ingestion period, Day 29 to Day 34 correspond to the third DSS ingestion period, and Day 50 to Day 56 correspond to the fourth DSS ingestion period. As is apparent from Fig. 3, the first episode of DSS-induced colitis was inhibited, and moreover, the subsequent three recurrences of DSS-induced colitis were inhibited and ameliorated by only single-dose administration of the anti-CD81 antibody on Day 0, whereby it exhibited a long-term remission-maintaining effect.

### Example 4

### Relapse-inhibitory effect of single-dose administration of an anti-CD81 antibody in the mouse model of colitis induced by an aqueous solution of dextran sulfate (DSS)

The anti-CD81 antibody clone 2F7 or clone Eat2 was administered once to the mouse model of relapse-remitting type ulcerative colitis, which is obtained by administering an aqueous solution of dextran sulfate (DSS) to a mouse, and the relapse-inhibitory effect of each clone of the anti-CD81 antibody on relapse-remitting type ulcerative colitis that was induced multiple times was examined.

### 1. Method

### (1) Preparation of D S S

Dextran sulfate (the product of Wako Pure Chemical Industries, Ltd., an average molecular weight of 5,000) was dissolved in drinking water to prepare a 2% aqueous solution.

### (2) Preparation of an administration solution

As the test drug to be studied, two kinds of hamster anti-CD81 antibodies were used, and as the pathological condition control test drug, hamster IgG was used.
The concentrated original solutions of the hamster anti-CD81 antibodies (Clone: 2F7, the product of SouthernBiotech or Clone: Eat2, the product of BioLegend, Inc., both of which were the anti-mouse CD81 monoclonal antibodies) were used as the administration solution, while phosphate buffer was added to the original solution of hamster IgG to prepare a 0.1 mg/ml solution.

### (3) Grouping, DSS ingestion, and drug administration

Four-week-old mice (BALB/cAnNCrlCrlj, male, the product of Charles River Laboratories Japan, Inc.) were purchased. The mice were reared for quarantine for seven days and then measured for body weight, and divided into four groups, where one group contained 15 mice. On the day of grouping, hamster IgG_{,} the anti-CD81 antibody clone 2F7 or the anti-CD81 antibody clone Eat2 were intraperitoneally administered once at 0.2 mg/mouse. The mice were fed 2% DSS ad libitum (first DSS ingestion) for five days after the grouping, and then normal drinking water for the next 10 days. Subsequently, the mice were fed 2% DSS ad libitum (second DSS ingestion) for five days, and then normal drinking water for the next 10 days. Subsequently, the mice were fed 2% DSS ad libitum (third DSS ingestion) for five days, and then normal drinking water for the next six days. The normal group was fed normal drinking water throughout the experimental period.

### (4) Colitis symptom score assessment

The symptoms of colitis were observed for 40 days from the day of grouping. The symptoms of colitis were assessed by score according to the condition of stool (normal stool (score: 0), loose stool (score: 1), and diarrhea (score: 2)).

### 2. Results

The chronological changes in the symptoms of colitis by colitis symptom score assessment in the mice that received single-dose administration of hamster IgG or either one of the hamster anti-CD81 antibody clone 2F7 and the anti-CD81 antibody clone Eat2 were shown in Fig. 4. From Fig. 4, it was observed that the first episode of DSS-induced colitis was inhibited, and further, the subsequent two recurrences of DSS-induced colitis were inhibited by only single-dose administration of the anti-CD81 antibody clone 2F7 or the anti-CD81 antibody clone Eat2 on Day 0, whereby it exhibited a long-term remission-maintaining effect.
Based on the results of the chronological changes in the symptoms of colitis in Fig. 4, an average colitis score of each of the mice from Day 0 to Day 11 was used as the first episode colitis score, an average colitis score of each of the mice from Day 15 to Day 23 was used as the relapse colitis score, and an average colitis score of each of the mice from Day 30 to Day 38 was used as the second relapse colitis score. Then, a two group comparison was performed between each group and the pathological condition control group (hamster IgG administration group) with respect to the colitis score (first episode colitis score, relapse colitis score, and second relapse colitis score) by the Wilcoxon test. The results thus obtained were shown in Table 4.

[Table 4]

**Table 4: Results of the colitis score assessment**

| Group | Symptoms of colitis(SEM) | | |
|---|---|---|---|
| | First episode colitis score | Relapse colitis score | Second relapse colitis score |
| | (Day 0-11) | (Day 15-23) | (Day 30-38) |
| Pathological condition control | 0.38 (0.08) | 0.64 (0.08) | 0.55 (0.09) |
| Single-dose administration of the anti-CD81 antibody 2F7 (0.2 mg/head) | 0.11 (0.03)* | 0.31 (0.09)* | 0.21 (0.06)* |
| Single-dose administration of the anti-CD81 antibody Eat2 (0.2 mg/head) | 0.04 (0.02)** | 0.23 (0.05)** | 0.21 (0.07)* |
| Normal mouse | 0.00 (0.00) ** | 0.00 (0.00)** | 0.00 (0.00)** |

| | | | |
|---|---|---|---|
| *;0.01<p<0.05,**;p<0.01 | | | |

From the results of Table 4, it was revealed that the persistent ameliorating effect on the symptoms of intestinal inflammation was obtained after single-dose administration of the anti-CD81 antibody (0.2 mg/head) as evidenced by the first episode colitis score, relapse colitis score, and second relapse colitis score.

### Industrial Applicability

The present invention revealed that refractory inflammatory bowel disease could be prevented, ameliorated, and treated by administration of an anti-CD81 antibody, and an anti-CD81 antibody had a long-term remission-maintaining effect even by single-dose administration. In other words, the present invention revealed that an anti-CD81 antibody exerted a profound effect even in a patient with refractory inflammatory bowel disease (IBD) in whom single-dose administration or even multiple-dose administration of an existing therapeutic agent for inflammatory bowel disease has achieved only as far as a remission-inducing effect.
Accordingly, a drug containing an anti-CD81 antibody as the active ingredient exhibits not only a remission-inducing effect on inflammatory bowel disease, but also a long-term remission-maintaining effect even by single-dose administration, and thus is utilizable as a drug for maintaining remission of inflammatory bowel disease and as a preventive, ameliorating, or therapeutic drug for inflammatory bowel disease.

## Claims

1. A drug for maintaining remission of inflammatory bowel disease, comprising an anti-CD81 antibody as an active ingredient.

2. A preventive, ameliorating, or therapeutic drug for refractory inflammatory bowel disease, comprising an anti-CD81 antibody as an active ingredient.

3. The preventive, ameliorating, or therapeutic drug according to claim 2, wherein the refractory inflammatory bowel disease refers to inflammatory bowel disease with repeated relapse or recurrence or inflammatory bowel disease with chronically persistent disease activity.

4. The preventive, ameliorating, or therapeutic drug according to claim 2 or 3, wherein the refractory inflammatory bowel disease is a steroid-dependent or steroid-resistant inflammatory disease.

5. The preventive, ameliorating, or therapeutic drug according to any one of claims 2 to 4, wherein the refractory inflammatory bowel disease refers to inflammatory bowel disease that is resistant or tolerant to usual treatment.

6. The preventive, ameliorating, or therapeutic drug according to claim 5, wherein the usual treatment refers to administration of an existing therapeutic drug.

7. The preventive, ameliorating, or therapeutic drug according to claim 6, wherein the existing therapeutic drug is a 5-aminosalicylic acid preparation, steroid, an immunosuppressant, a TNF inhibitor, or an integrin inhibitor.

8. The preventive, ameliorating, or therapeutic drug according to claim 7, wherein the existing therapeutic drug is sulfasalazine, an anti-TNE antibody, salazosulfapyridine, mesalazine, betamethasone, betamethasone sodium phosphate, betamethasone phosphate, prednisolone, azathioprine, tacrolimus, 6-mercaptopurine, cyclosporine, infliximab, adalimumab, certolizumab, pegol, or natalizumab.

9. The drug according to any one of claims 1 to 8, wherein the drug has a long-term remission-maintaining effect by single-dose administration.

10. The drug according to claim 9, wherein the remission-maintaining effect is an effect of maintaining a state of remission by inhibiting repetition of relapse or recurrence of intestinal inflammation.

11. The drug according to any one of claims 1 to 4, wherein the drug is administered to a patient with inflammatory bowel disease in whom remission cannot be maintained by single-dose or even by multiple-dose administration of an existing therapeutic drug.

12. The drug according to claim 11, wherein the existing drug is the existing drug according to claim 7 or 8.

13. The drug according to any one of claims 1 to 12, wherein the drug is administered once every 2 to 30 weeks during a period of treatment.

14. The drug according to claim 13, wherein a dose per administration is 0.01 to 15 mg/kg.

15. The drug according to any one of claims 1 to 14, wherein the inflammatory bowel disease is ulcerative colitis.

16. The drug according to any one of claims 1 to 14, wherein the inflammatory bowel disease is Crohn's disease.
